# EUROPÄISCHE PATENTSCHRIFT

(11) **EP 2 993 221 B1**
(45) Veröffentlichungstag und Bekanntmachung des Hinweises auf die Patenterteilung: **09.01.2019**
(21) Anmeldenummer: 14183998.5
(22) Anmeldetag: 08.09.2014
(51) Int. Cl.: C11B 9/00, A61Q 13/00, C11D 3/50, C11D 17/00, A61Q 19/00, A61K 8/84, A61Q 19/10, A61K 8/11

(54) **Verkapselte Duftstoffmischungen**
Encapsulated fragrance mixtures
Mixtures de parfums capsulées

(43) Veröffentlichungstag der Anmeldung: 09.03.2016
(73) Patentinhaber: Symrise AG, 37603 Holzminden (DE)
(72) Erfinder: Finke, Anja, 37603 Holzminden (DE); Kulke, Torsten, 37671 Höxter (DE); Siegel, Sven, 37671 Höxter (DE); Ott, Patrick, 37603 Holzminden (DE)
(74) Vertreter: Fabry, Bernd

(56) Entgegenhaltungen:
- EP-A1- 1 767 185
- EP-A1- 1 894 603
- EP-A1- 2 204 155
- EP-A2- 2 620 211
- WO-A1-2004/015050
- WO-A1-2013/000842
- WO-A1-2014/187833
- WO-A1-2015/117893
- WO-A1-2015/165582
- WO-A2-2010/142815
- WO-A2-2013/109798
- DE-A1-102012 221 619
- US-A1- 2010 137 178
- US-A1- 2014 161 740

## Beschreibung

### GEBIET DER ERFINDUNG

Die Erfindung befindet sich auf dem Gebiet von Duftstoffen und Duftstoffmischungen daraus.

### STAND DER TECHNIK

In der Parfümindustrie besteht generell ein ständiger Bedarf an neuen Duftstoffmischungen, da den Konsumenten laufend neue und moderne Düfte mit frischen Duftnoten zur Verfügung gestellt werden sollen. Duftstoffmischungen mit den unterschiedlichsten Duftnoten werden in großen Mengen und ungezählten Variationen in Parfüms, Riechstoffmischungen (Parfümkompositionen) und Parfümierungen für die verschiedensten Anwendungsgebiete eingesetzt. Wegen der steigenden Nachfrage der Verbraucher nach immer neuen Duftstoffmischungen mit neuen Duftnoten, besteht in der Parfümindustrie ein ständiger Bedarf an neuen Duftstoffmischungen, die neuartige Effekte erzielen, so dass sich auf diese Art neue Modetrends kreieren lassen.

Trotz einer Vielzahl bereits vorhandener Duftstoffmischungen, besteht in der Parfümindustrie für Kreation neuartiger moderner Parfümkompositionen ein ständiger Bedarf an neuen Duftstoffmischungen mit besonderen geruchlichen Eigenschaften, die geeignet sind, als Grundlage für die Komposition von neuartigen modernen Parfüms mit komplexem Charakter zu dienen. Insbesondere ist der Fokus bei neuen Duftstoffmischungen darüber hinaus darauf gerichtet, dass sie über Ihre primären, nämlich geruchlichen, Eigenschaften hinaus, zusätzliche positive Sekundäreigenschaften besitzen, wie z.B. eine höhere Stabilität unter bestimmten Anwendungsbedingungen, eine hohe Ausgiebigkeit, eine große Strahlungskraft, gute Diffusivität (d.h. eine gute Raumwirkung), Fülle, Kraft und/oder Natürlichkeit, geruchsverstärkende Eigenschaften oder aber auch bessere dermatologische Verträglichkeit, gute Löslichkeit, sowie die toxikologische Unbedenklichkeit und biologische Abbaubarkeit.

Kombinationen von Duftstoffen, die zusammen eine Duftstoffmischung ergeben, welche den Verbraucher gefallen und die, die oben genannten Sekundäreigenschaften besitzen, sind stets schwer zu identifizieren, da zum einen die Mechanismen der Geruchswahrnehmung nicht ausreichend bekannt ist, und auch die Zusammenhänge zwischen der speziellen Geruchswahrnehmung einerseits und der chemischen Struktur des zugehörigen Riechstoffs andererseits nicht hinreichend erforscht sind, so dass häufig bereits geringfügige Änderungen am strukturellen Aufbau eines bekannten Riechstoffs zu starken Änderungen der sensorischen Eigenschaften und Beeinträchtigungen der Verträglichkeit für den menschlichen Organismus, bewirken.

Es besteht daher in der Parfümindustrie grundsätzlich ein ständiger Bedarf an neuen Duftstoffmischungen, die sich zur Herstellung von Riechstoffkompositionen oder parfümierten Artikeln eignen. Insbesondere besteht ein Bedarf an Duftstoffmischungen, die durch die oben erwähnten technischen Eigenschaften zu einem erhöhten Nutzen von Riechstoffkompositionen und Parfümölen führen.

Die Aufgabe der vorliegenden Erfindung hat daher darin bestanden, einzelne Duftstoffe und Aromastoffe zu detektieren, die besonders geeignet sind zu Duftstoffmischungen zusammengemischt zu werden. Es war insbesondere Aufgabe der vorliegenden Erfindung eine generelle Definition für Duftstoffe bzw. Aromastoffe zu finden, die zu stabilen Duftstoffmischungen zusammengemischt werden können und sich im Anschluss ohne große Probleme verkapseln lassen. Insbesondere sollen die neuen Duftstoffmischungen derart gestaltet sein, dass sie sich durch die verschiedensten Verkapselungsprozesse, welche im Stand der Technik bekannt sind, mit den verschiedensten Trägerstoffen verkapseln lassen, ohne dass dabei große oder wenn, dann nur geringe Verluste der Duftstoffe durch Diffusion oder dergleichen bei der Verkapselung im wässrigen Medien bzw. bei der Trocknung auftreten. Zudem sollen die so eingekapselten Duftstoffmischungen einen langanhaltenden Duft und einen hohen Impact aufweisen, d.h. dass eine schnelle Geruchswahrnehmung nach dem Aufbrechen oder Lösen der Kapseln erfolgt.

### BESCHREIBUNG DER ERFINDUNG

Gegenstand der Erfindung sind daher Duftstoffmischungen, umfassend mindestens 60 Gew.-%, vorzugsweise mindestens 70 Gew.-%, besonders bevorzugt mindestens 80 Gew.-% Duftstoffe, die wenn sie entlang einer virtuellen Achse X, Y und Z ausgerichtet sind, eine Abmessung von X > 5 Angström, Y > 3 Angström und Z > 2 Angström aufweisen, bevorzugt weisen die Aroma- und / oder Duftstoffmoleküle eine Abmessung von X > 7 Angström, Y > 4 Angström und Z > 2 Angström auf, wobei die Anzahl der so definierten verschiedenen Duftstoffen in der Duftstoffmischung mindestens zwei beträgt und die mindestens zwei Duftstoffe ausgewählt sind aus der Gruppe bestehend aus Agrunitril, Buccoblätteröl, Thiocineol, 2-Isobutyl-3-methoxypyrazin, Rosenoxid, Aldehyd C16, Methyloctincarbonat, Precyclemone B (CAS 52474-60-9), Allylamylglycolat, Cis-4-Decenal sowie deren Mischungen, wobei mindestens einer der Duftstoffe in der Duftstoffmischung ein Schwefel- oder Stickstoffatom aufweist..

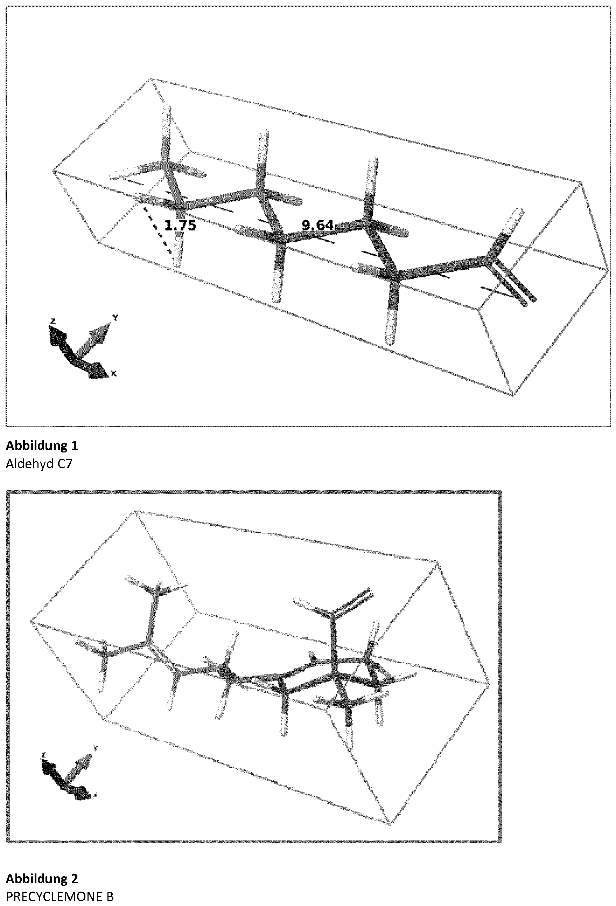

Bei der Detektion von besonders geeigneten Duftstoffen für Duftstoffmischungen und anschließender Verkapselung von diesen, wurden die Strukturen von einzelnen Duftstoffmolekülen unter Berücksichtigung ihrer Stereochemie per Kraftfeld energieminimiert und zur weiteren Berechnung entlang einer virtuellen Achse X, Y und Z ausgerichtet. Anschließend wurde um die jeweiligen Strukturen eine virtuelle Box erzeugt und die Ausdehnung der Box wurde zur weiteren Berechnung übernommen (siehe Abbildung 1 und 2). Überraschenderweise wurde gefunden, dass die so detektierten Duftstoffe besonders gut geeignet sind zu Duftstoffmischungen kombiniert und zusammengemischt zu werden und besonders vorteilhaft sind, da sie sich anschließend problemlos verkapseln lassen.

Bevorzugt umfasst eine erfindungsgemäße Duftstoffmischung daher mindestens 80 Gew.-% Duftstoffe, die, wenn sie entlang einer virtuellen Achse X, Y und Z ausgerichtet vorlägen, eine Abmessung von X > 7 Angström, Y > 4 Angström und Z > 2 Angström aufweisen.

In einer bevorzugten Ausführungsform hat sich gezeigt, dass es besonders vorteilhaft ist, wenn die einzelnen Duftstoffe in der erfindungsgemäßen Duftstoffmischung einen Geruchsschwellenwert (GSW) von kleiner als 10 ppm (in Luft), vorzugsweise von kleiner als 5 ppm, besonders bevorzugt von kleiner als 2 ppm, aufweist.

Die Geruchsschwelle ist die Schwelle, an der ein Duftstoff oder Riechstoff von einem Organismus olfaktorisch wahrgenommen wird. Der Geruchsschwellenwert, kurz GSW, ist jene minimale Konzentration eines bestimmten gasförmigen, sensorisch aktiven Stoffes im umgebenden Medium, die dieses Lebewesen durch seinen Geruchssinn gerade noch wahrnehmen kann. In der vorliegenden Erfindung sind Aroma- und Duftstoffe bevorzugt, die einen (sehr) niedrigen Geruchsschwellenwert aufweisen. Je kleiner dieser ist, desto geringer ist die Konzentration bei der, der Duft dann wahrgenommen wird.

In einer weiteren bevorzugten Ausführungsform der vorliegenden Erfindung ist die Anzahl an verschiedenen Duftstoffen in einer erfindungsgemäßen Duftstoffmischung bei höchstens gleich oder unter 10. Vorzugsweise weist eine erfindungsgemäße Duftstoffmischung drei, vier, fünf, sechs, sieben, acht oder neun unterschiedliche Duftstoffe auf. Bevorzugt sind Duftstoffmischungen mit 5, 6, 7 oder 8 unterschiedlichen Duftstoffen, ganz besonders bevorzugt sind Mischungen mit 6, 7 oder 8 unterschiedlichen Duftstoffen.

Weiterhin liegt in einer bevorzugten Ausführung in der erfindungsgemäßen Duftstoffmischung mindestens einer der Duftstoffe in einer Konzentration von mindestens 10 Gew.-%, bevorzugt mindestens 15 Gew.-%, besonders bevorzugt mindestens 20 Gew.-% und vorzugsweise höchstens bei 60 Gew.-%, besonders bevorzugt höchstens bei 50 Gew.-%, in der Zusammensetzung vor. Vorzugsweise liegt die Konzentration von mindestens zwei der Duftstoffe bei mindestens 15 Gew.-%.

In einer erfindungsgemäßen Duftstoffmischung weist mindestens einer der Duftstoffe ein Schwefel- oder Stickstoffatom auf, vorzugsweise handelt es sich hierbei um eine Nitrilgruppe.

Weiterhin ist es in einer erfindungsgemäßen Duftstoffmischung von Vorteil, wenn mindestens einer der Duftstoffe ein Molgewicht von größer als 120 g/mol aufweist.

In einer weiteren bevorzugten Ausführungsform weisen die einzelnen Duftstoffe der erfindungsgemäßen Duftstoffmischung einen log K_{ow} Wert von 1 bis 10, vorzugsweise von 1.5 bis 8, besonders bevorzugt von 2 bis 5.5 auf.

Der log K_{ow} (*Octanol*/*Wasser*-*Verteilungskoeffizient*) ist ein Maß für die Lipophilie einer Substanz. Rechnerisch ist es der Verteilungskoeffizient K_{ow} einer Substanz in einem Gemisch von 1-Octanol und Wasser. *K*_{ow} ist größer als eins, wenn eine Substanz besser in fettähnlichen Lösungsmitteln wie *n*-Octanol löslich ist, kleiner als eins wenn sie besser in Wasser löslich ist. Entsprechend ist Log K_{ow} positiv für lipophile und negativ für hydrophile Substanzen.

Das besondere an der vorliegenden Erfindung ist die Kombination aus den unterschiedlichen, oben genannten Parametern für ein Duftstoffmolekül. Duftstoffe, die die oben genannten Parameter aufweisen lassen sich besonders gut miteinander zu einer Duftstoffmischung kombinieren, die Vorteile aufzeigen, wie gute Stabilität, gute Verkapselungseigenschaften sowie die Notwendigkeit von weiteren Lösungsmitteln minimiert.

Es zeigte sich, dass die Duftstoffmischungen der vorliegenden Erfindungen auch ohne Lösungsmittel formuliert und verkapselt werden können.

Demgemäß enthält in einer bevorzugten Ausführungsform die erfindungsgemäße Duftstoffmischung kein oder nur sehr wenig Lösungsmittel.

Unter "sehr wenig Lösungsmittel" ist demgemäß ein Gehalt von kleiner als 30 Gew.-%, bevorzugt kleiner als 20 Gew.-% Lösungsmittel in einer erfindungsgemäßen Duftstoffmischung zu verstehen.

Überraschenderweise wurde auch wie bereits erwähnt gefunden, dass die erfindungsgemäßen Duftstoffmischungen der vorliegenden Erfindung besonders vorteilhaft sind, da sie sich problemlos in verschiedensten Verkapselungsprozessen unter Verwendung von verschiedensten Trägerstoffen, verkapseln lassen. Insbesondere zeigte sich, dass die erfindungsgemäßen Duftstoffmischungen bei der Verkapselung in wässrigen Medien nur geringe oder gar keine Verluste durch Diffusion aufweisen. Ebenso zeigte es sich, dass bei der Trocknung der Kapseln die Duftstoffe kaum bis geringfügig verlorengehen.

Daher ist ein weiterer Gegenstand der vorliegenden Erfindung Kapseln, enthaltend die erfindungsgemäßen Duftstoffmischungen der oben genannten Art.

Die Kapseln weisen vorzugsweise einen durchschnittlichen Durchmesser von 1 µm bis 8 mm aufweisen, bevorzugt 5 µm bis 2mm, besonders bevorzugt 10 µm bis 500 µm. Die Kapselgrößen sind nicht beschränkt und können je nach Anwendungs- und Einsatzgebiet nach Bedarf entsprechend variabel hergestellt werden.

Demgemäß in können einer bevorzugten Ausführung die Kapseln, je nach Anwendungsbedarf, einen beliebigen durchschnittlichen Durchmesser von zwischen 1 µm bis 8 mm annehmen.

### VERKAPSELUNG

Verkapselungstechniken von Duftstoffen sind aus dem Stand der Technik bekannt. Üblicherweise erfolgen Verkapselungen durch beispielsweise Verfahren wie Extrusion, Einbettung in z.B. Gelatinekapseln, Sprühtrocknung, Agglomeration (Nassgranulation in Mischern bzw. Wirbelschichtagglomeration), oder durch Wirbelschichtsprühgranulation. Ein weiteres mögliches Verfahren zur Herstellung zur Verkapselung stellt die Aufbaugranulation in der Wirbelschicht dar, wie es in DE 199 56 604 A1 veröffentlicht wurde. Hier wird ein Prozess dargestellt bei dem die flüssige Formulierung in eine Wirbelschicht durch Sprühdüsen eingedüst wird. Das Verfahren wendet einen Wirbelschicht-Prozess gemäß EP-A-0163836 und EP-A-0332929 an.

Derartige Verfahren sind wohlbekannt und erzeugen Duftstoffkapseln mit definierten Produktqualitäten. Diese Aroma- bzw. Duftstoffkapseln werden in einer Vielzahl von Industriezweigen in immer größerem Umfang genutzt. Dies betrifft sowohl die hergestellten Mengen als auch die verschiedensten Formulierungen.

Üblicherweise erfolgen Verkapselungen mit Hilfe von festen Überzugsmaterialien, wie beispielsweise Stärken, einschließlich deren Abbauprodukten sowie chemisch oder physikalisch erzeugten Derivaten (insbesondere Dextrine und Maltodextrine), Gelatine, Gummi Arabicum, Agar-Agar, Ghatti Gum, Gellan Gum, modifizierte und nicht-modifizierte Cellulosen, Pullulan, Curdlan, Carrageenane, Alginsäure, Alginate, Pektin, Inulin, Xanthan Gum und Mischungen von zwei oder mehreren dieser Substanzen.

Das feste Verkapselungsmaterial ist vorzugsweise eine Gelatine (insbesondere Schweine-, Rind-, Geflügel- und/oder Fischgelatine), wobei diese vorzugsweise einen Schwellfaktor von größer oder gleich 20, vorzugsweise von größer oder gleich 24 aufweist. Ebenfalls bevorzugt sind Maltodextrine (insbesondere auf Basis von Getreide, speziell Mais, Weizen, Tapioka oder Kartoffeln), die vorzugsweise DE-Werte im Bereich von 10 bis 20 aufweisen. Weiterhin bevorzugt sind Cellulosen (z.B. Celluloseether), Alginate (z.B. Natriumalginat), Carrageenan (z.B. beta-, jota-, lambda- und/oder kappa-Carrageenan), Gummi Arabicum, Curdlan und/oder Agar Agar. Entsprechende Kapseln werden beispielsweise in den folgenden Schriften ausführlich EP 0389700 A1**,** US 4,251,195**,** US 6,214,376**,** WO 2003 055587 oder WO 2004 050069 A1**.**

Die Kapseln können alternativ auch in Form von Mikrokapseln vorliegen. Unter den Begriffen "Mikrokapsel" oder "Nanokapsel" werden vom Fachmann sphärische Aggregate mit einem Durchmesser im Bereich von etwa 0,0001 bis etwa 5 und vorzugsweise 0,005 bis 0,5 mm verstanden, die mindestens einen festen oder flüssigen Kern enthalten, der von mindestens einer kontinuierlichen Hülle umschlossen ist. Genauer gesagt handelt es sich um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen. Nach einem anderen Verfahren werden geschmolzene Wachse in einer Matrix aufgenommen ("microsponge"), die als Mikropartikel zusätzlich mit filmbildenden Polymeren umhüllt sein können. Nach einem dritten Verfahren werden Partikel abwechselnd mit Polyelektrolyten unterschiedlicher Ladung beschichtet ("layer-by-layer"-Verfahren). Die mikroskopisch kleinen Kapseln lassen sich wie Pulver trocknen. Neben einkernigen Mikrokapseln sind auch mehrkernige Aggregate, auch Mikrosphären genannt, bekannt, die zwei oder mehr Kerne im kontinuierlichen Hüllmaterial verteilt enthalten. Ein- oder mehrkernige Mikrokapseln können zudem von einer zusätzlichen zweiten, dritten etc. Hülle umschlossen sein. Die Hülle kann aus natürlichen, halbsynthetischen oder synthetischen Materialien bestehen. Natürlich Hüllmaterialien sind beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccharide, wie Stärke oder Dextran, Polypeptide, Proteinhydrolysate, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Aminoplaste, Phenolharze, Polyharnstoffe, Polyacrylate, Polyamide, Polyvinylalkohol oder Polyvinylpyrrolidon.

Beispiele für Mikrokapseln des Stands der Technik sind folgende Handelsprodukte (in Klammern angegeben ist jeweils das Hüllmaterial) : *Hallcrest Microcapsules* (Gelatine, Gummi Arabicum), *Coletica Thalaspheres* (maritimes Collagen), *Lipotec Millicapseln* (Alginsäure, Agar-Agar), *Induchem Unispheres* (Lactose, mikrokristalline Cellulose, Hydroxypropy-Imethylcellulose); *Unicerin C30* (Lactose, mikrokristalline Cellulose, Hydroxypropylmethylcellulose), *Kobo Glycospheres* (modifizierte Stärke, Fettsäureester, Phospholipide), *Softspheres*

(modifiziertes Agar-Agar) und *Kuhs Probiol Nanospheres* (Phospholipide) sowie *Primaspheres* und *Primasponges* (Chitosan, Alginate) und *Primasys* (Phospholipide).

Eine besonders bevorzugte Form der Verkapselung besteht darin, die Duftstoffe in eine Matrix einzubringen und diese dann mit einer Schale zu umgeben. Hierzu kennt der Stand der Technik eine ganze Reihe von verfahren.

Ein Beispiel stellen so genannte Chitosanmikrokapseln dar, deren Herstellverfahren aus dem Stand der Technik hinlänglich bekannt ist **[**WO 01/01926**,** WO 01/01927**,** WO 01/01928**,** WO 01/01929**].** Mikrokapseln mit mittleren Durchmessern im Bereich von 0,0001 bis 5, vorzugsweise 0,001 bis 0,5 und insbesondere 0,005 bis 0,1 mm, bestehend aus einer Hüllmembran und einer die Wirkstoffe enthaltenden Matrix, können beispielsweise erhalten werden, indem man
- aus Gelbildnern, kationischen Polymeren und Wirkstoffen eine Matrix zubereitet,
- gegebenenfalls die Matrix in einer Ölphase dispergiert,
- die dispergierte Matrix mit wässrigen Lösungen anionischer Polymere behandelt und gegebenenfalls dabei die Ölphase entfernt.

Die Schritte (a) und (c) sind dabei insofern austauschbar, als man anstelle der kationischen Polymeren in Schritt (a) anionische Polymere einsetzt und umgekehrt.

Man kann Kapseln auch erzeugen, indem man den Wirkstoff abwechselnd mit Schichten aus unterschiedlich geladenen Polyelektrolyten einhüllt (layer-by-layer-Technologie). In diesem Zusammenhang sei auf das Europäische Patent EP 1064088 B1 (Max-Planck Gesellschaft) verwiesen.

Oftmals ist es bei derart verkapselten Aroma- bzw. Duftstoffe so, dass diese erst verzögert oder durch einen externen Auslöser (z.B. Wasser, Scherkräfte, pH-Wertänderung) freigesetzt werden, so dass die Aroma- bzw. Duftstoffwahrnehmung ("Impact") in den ersten Sekunden oder vor Einwirken des Auslösers gering ist.

Es zeigte sich, dass die erfindungsgemäßen eingekapselten Duftstoffmischungen einen hohen Impact aufzeigten, d.h. das Aroma bzw. der Duft stark wahrgenommen werden konnte, so dass ein erster starker Dufteindruck erzeugt wird.

Es zeigte sich zudem überraschenderweise, dass Kapseln enthaltend die erfindungsgemäßen Duftstoffmischungen in klaren Anwendungen eingearbeitet werden konnte, und es dabei zu keiner Trübung der Formulierung führte. Vorteilhafterweise diffundieren die erfindungsgemäßen Duftstoffmischungen in wässrigen Medien, insbesondere solche die Tenside oder Emulgatoren enthalten, nicht aus der Kapsel, so dass diese einigermaßen stabil sind.

Es zeigte sich auch, dass die so eingekapselten Duftstoffmischungen in harschen Umgebungen in Gegenwart von beispielsweise Oxidationsmitteln, Bleichmitteln, in hohe bzw. niedriger pH- Wert Umgebung, stabil blieben. Dadurch sind die erfindungsgemäßen Duftstoffmischungen besonders geeignet, um in Produkten eingearbeitet zu werden, in denen solche Bedingungen vorliegen, beispielsweise in Wasch- und Reinigungsmittel oder Kosmetikprodukten.

Demgemäß ist ein weiterer Aspekt der vorliegenden Erfindung Konsumprodukte umfassend die erfindungsgemäßen Duftstoffmischungen oder Kapseln enthaltend die erfindungsgemäße Duftstoffmischung.

### GEWERBLICHE ANWENDBARKEIT

Ein weiterer Aspekt der vorliegenden Erfindung ist die Anwendung der erfindungsgemäßen Duftstoffmischung in Konsumprodukten.

Demgemäß ist ein weiterer Gegenstand der vorliegenden Erfindung Konsumprodukte, umfassend eine erfindungsgemäße Duftstoffmischung und / oder Kapseln, enthaltend die erfindungsgemäße Duftstoffmischung. Hierbei sind Konsumprodukte im Rahmen der vorliegenden Erfindung, unterschiedliche Produkte, wie beispielsweise aus den Bereichen Wasch- und Reinigungsmittel, kosmetische Produkte, Parfümartikel sowie kosmetische Reinigungsmittel, die vorzugsweise ausgewählt sind aus der Gruppe bestehend aus:

Parfüm-Extraits, Eau de Parfums, Eau de Toilettes, Rasierwässer, Eau de Colognes, Pre-shave-Produkte, Splash-Colognes, parfümierte Erfrischungstücher, Parfüms für saure, alkalische und neutrale Reinigungsmittel, Waschmittel, Waschtabletten, Desinfektionsmitteln, sowie von Luftverbesserern, Aerosolsprays, Wachse und Polituren, sowie Körperpflegemit-teln, Badeölen, kosmetischen Emulsionen, wie z.B. Hautcremes- und -lotionen, Sonnenschutzcremes- und -lotionen, After-sun-cremes und -lotionen, Handcremes und - lotionen, Fußcremes und -lotionen, Enthaarungscremes und -lotionen, After-shave-Cremes und -lotionen, Bräunungscremes und -lotionen, Haarpflegeprodukten wie z.B. Haarsprays, Haar-gelen, festigende Haarlotionen, Haarspülungen, Haarfärbemitteln, Haarverformungsmitteln und Haarglättungsmitteln, Haarwässern, Haarcremes und -lotionen, Deodorantien und Antiperspirantien, Produkten der dekorativen Kosmetik wie z.B. Lidschatten, Nagellacke, Make-ups, Lippenstifte, Mascara sowie von Kerzen, Lampenölen, Räucherstäbchen, Insekti-ziden, Repellentien, Treibstoffen.

Erfindungsgemäße Duftstoffmischungen bzw. Kapseln, die diese enthalten können allgemein (z.B. in konzentrierter Form, in Lösungen oder in unten beschriebener modifizierter Form) verwendet werden für die Herstellung von z.B. Parfüm-Extraits, Eau de Parfums, Eau de Toilettes, Rasierwässer, Eau de Colognes, Pre-shave-Produkte, Splash-Colognes und parfümierten Erfrischungstüchern sowie die Parfümierung von sauren, alkalischen und neutralen Reinigungsmitteln, wie z.B. Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z.B. festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-ÖI-in-Wasser-Typ wie z.B. Hautcremes- und -lotionen, Gesichtscremes und -lotionen, Sonnenschutzcremes und lotionen, After-sun-cremes und -lotionen, Handcremes und -lotionen, Fußcremes und lotionen, Enthaarungscremes und -lotionen, After-shave-Cremes und -lotionen, Bräunungscremes und -lotionen, Haarpflegeprodukten wie z.B. Haarsprays, Haargelen, festigen Haarlotionen, Haarspülungen, permanenten und semipermanenten Haarfärbemitteln, Haarverformungsmitteln wie Kaltwellen und Haarglättungsmitteln, Haar-wässern, Haarcremes und - lotionen, Deodorantien und Antiperspirantien wie z.B. Achsel-sprays, Roll-ons, Deosticks, Deocremes, Produkten der dekorativen Kosmetik wie z.B. Lid-schatten, Nagellacke, Make-ups, Lippenstifte, Mascara sowie von Kerzen, Lampenölen, Räucherstäbchen, Insektiziden, Repellentien, Treibstoffen.

### BEISPIELE

### BEISPIELE 1 BIS 10, VERGLEICHSBEISPIELE V1 BIS V3

### BERECHNUNGEN DER DUFTSTOFFMOLEKÜLE

Die Strukturen von einzelnen Duftstoffmoleküle wurden unter Berücksichtigung der Stereochemie, soweit sicher bekannt, per Kraftfeld energieminimiert und zur weiteren Berechnung entlang einer virtuellen Achse ausgerichtet. Anschließend wurde um die jeweiligen Strukturen eine virtuelle Box erzeugt und die Ausdehnung der Box wurde zur weiteren Berechnung übernommen. (Maßeinheit: Angström). Die Beispiel 1 bis 10 sind erfindungsgemäß, die Beispiel V1 bis V3 dienen zum Vergleich.
Software:

| | |
|---|---|
| Fa. Schrödinger LLC | |
| Maestro | Version 9.5.014 |
| Modul: | MacroModel |
| Kraftfeld: | OPLS2005 |
| Parameter: | Gasphase, Constant dielectric |

**Tabelle 1**

| Daten zu den berechneten Duftstoffe, die erfindungsgemäß einsetzbar sind | | | | | | | | |
|---|---|---|---|---|---|---|---|---|
| **Bsp** | **Verbindung** | **Struktur** | **Abmessung** [Angström] | | | **GWS** | **log Kow** | **Gewich t** |
| | | | x | y | z | ppm | | mol/ g |
| 1 | Rosenoxid (FEMA 3236) | 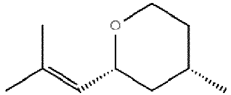 | 9.11 | 4.76 | 4.55 | 0.5 | 3.58 | 154,3 |
| 2 | Aledhyd C16 (FEMA 2444) | 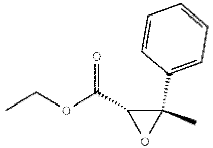 | 8.07 | 7.16 | 4.82 | 0.2 | 3 | 206,2 |
| 3 | Agrunitril (Citronellyl nitrile, CAS 51566-62-2) | 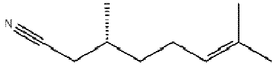 | 10.56 | 4.01 | 3.43 | 1 | 3.55 | 151,3 |
| 4 | Methyl-Octin-Carbonat (CAS 111-80-8) | 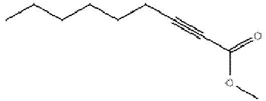 | 12.04 | 6.29 | 2.24 | 0.2 | 3.1 | 168,2 |
| 5 | Bucco-Blätteröl | 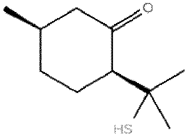 | 7.67 | 5.61 | 4.64 | 0.2 | | |
| 6 | Precyclemone B (CAS 52474-60-9) | 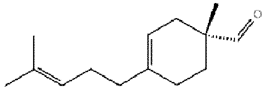 | 11.58 | 4.87 | 4.40 | 0.5 | 5.19 | 206,3 |
| 7 | Allylamylglycolat (CAS 67634-00-8) | 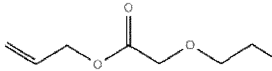 | 11.72 | 5.24 | 4.56 | 0.1 | 2.34 | 186,3 |
| 8 | Thiocineol (FEMA 4108) | 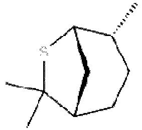 | 7.02 | 5.29 | 4.93 | <0.01 | 3.95 | 170,3 |
| 9 | 2-Isobutyl-3-methoxypyrazin (FEMA 3132) | 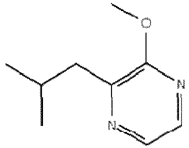 | 7.50 | 6.99 | 4.47 | 0.1 | 2.86 | 166,2 |
| 10 | Cis-4-Decenal (FEMA 3264) | 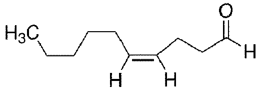 | 11.50 | 5.83 | 3.50 | 0.2 | 3.55 | 154,3 |
| V1 | Aldehyd C-7 (FEMA 2540) | 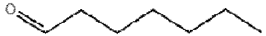 | 9.64 | 2.59 | 1.73 | | | 114,2 |
| V2 | Ethylbutyrat (FEMA2427) | 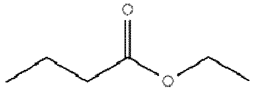 | 9.20 | 2.93 | 1.76 | | | 116,2 |
| V3 | Diphenyloxid (CAS 32576-61-7) | 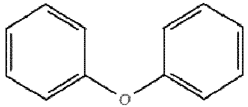 | 10.08 | 4.81 | 0.0 | | | 170,2 |

### ANWENDUNGSBEISPIELE 1: DUFTSTOFFMISCHUNGEN I BIS V

Die geeigneten Duftstoffe aus Tabelle 1 wurden in unterschiedlichen Formulierungen zu Duftstoffmischungen I bis V gemischt und anschließend verkapselt.

### Herstellung der Kapseln

In ein zylindrisch geformtes 1I-Rührgefäß mit eingebautem Rührorgan (Dissolver) wurden unter langsamem Rühren 200 g Wasser, 35 g einer 20 gew.-%igen wäßrigen Lösung eines Schutzkolloids, 40,5g einer 70 gew.-%igen wässrigen Lösung des Melamin-Formaldehydharzmischung (z.B. Luracoll SD ex. BASF) gegeben.
Nun wurde die Drehzahl des Dissolvers so stark erhöht, daß eine gute Durchmischung erzielt wurde. Immer noch unter Durchmischung wurden nun 200 g zu verkapselnden Duftölmischung langsam zugegeben. Es wurde auf 30-40 °C temperiert und die Dissolverdrehzahl auf 1200 U/Min gestellt. Anschließend wurden X g 10 gew.-%ige Ameisensäure zugegeben und die Dissolverdrehzahl auf 1000 U/Min verringert. Nach ungefähr 20 min. bildeten sich Kapseln. Sobald die gewünschte Kapselgröße erreicht war, wurde die Umdrehungszahl des Dissolvers auf 1000 U/Min gesenkt und die Kapseldispersion eine halbe Stunde bei 30 - 40°C und 1000 U/Min gerührt.

Die Ergebnisse sind in **Tabelle 2** zusammengefasst.

**Tabelle 2**

| Erfindungsgemäße Duftstoffmischungen I bis V | | | | | |
|---|---|---|---|---|---|
| **Duftstoffmischungen** | **I** | **II** | **III** | **IV** | **V** |
| **Verbindung** | | | | | |
| Rosenoxid | 50 | 50 | 50 | 50 | 100 |
| Aldehyd C16 | - | - | - | 450 | - |
| Agrunitril | 200 | 195 | 195 | 20 | 200 |
| Diphenyloxid | - | - | - | - | 2 |
| Bucco-Blätteröl | - | - | - | 2 | - |
| Precyclemone B | 300 | 280 | 290.5 | - | 260 |
| Allylamylglycolat | 400 | 380 | 392 | 450 | 400 |
| Thiocineol | - | 40 | 20 | 60 | |
| 2-Isobutyl-3-methoxy-pyrazin | - | 5 | 2.5 | - | 8 |
| Cis-4-Decenal | 50 | 50 | 50 | 18 | 30 |
| Gesamtmenge | 1000 | 1000 | 1000 | 1000 | 1000 |

### ANWENDUNGSBEISPIEL 2

### Stabilitätstest der verkapselten Duftstoffmischungen I bis V

Die entstandenen Kapseldispersionen wurden zur Bestimmung des freien, (nicht verkapselten) Ölgehaltes mit Aceton extrahiert und die Konzentration der Riechstoffmischungen in dem erhaltenen Extrakt bestimmt.

Zur Bestimmung des Gesamtölgehaltes wurden die entstandenen Kapseldispersionen erschöpfend mit einem geeigneten Lösungsmittel (z.B. Ethanol, Aceton) extrahiert. Dieser Extrakt wurde auf ein bekanntes Volumen aufgefüllt und der Gehalt der Riechstoffmischung bestimmt.

Die Ergebnisse sind in **Tabelle 3** zusammengefasst.

**Tabelle 3**

| Bestimmung der Kapselqualität (Alle Mengenangaben als Gew.-%) | | | |
|---|---|---|---|
| **Kapseln mit Duftstoffmischung** | **Freies Öl, nicht verkapselt*** | **Öl, Gesamtmenge** | **Gesamtgehalt an durchschnittlicher Duftstoffmenge in einer Kapsel** |
| I | 0.00 | 0.0 | 33.0 |
| II | 0.02 | 0.86 | 27.9 |
| III | 0.00 | 0.14 | 31.7 |
| IV | 0.00 | 0.16 | 34.5 |
| V | 0.00 | 0.00 | 33.7 |

### ANWENDUNGSBEISPIEL 3

### Stabilitätstest in Weichspülern

Eine handelsübliche, parfümfreie Weichspülerzubereitung mit ca. 15% Esterquatanteil wurde mit 0,5 g der verkapselten Duftstoffmischungen I bis V versetzt und bei 40 °C gelagert. Nach einer Lagerzeit von 1, 4, 8 und 12 Wochen wurde der Gehalt der in die Masse diffundierten Riechstoffe mit Hilfe von Headspace-messungen in der darüberstehenden Luftphase bestimmt. Die Ergebnisse sind in **Tabelle 4** zusammengefasst. Angegeben ist der prozentuale Anteil, des noch in der Kapsel verbliebenen Öls.

**Tabelle 4**

| Stabilitätsuntersuchung (Angaben als %-rel. Bezogen auf die Ausgangszubereitung) | | | | |
|---|---|---|---|---|
| **Kapseln mit Duftstoffmischung** | **Stabilität der Kapseln im Weichspüler bei 40 °C** | | | |
| | 1 Woche | 4 Wochen | 8 Wochen | 12 Wochen |
| I | 100 | 97 | 74 | 35 |
| II | 89 | 82 | 58 | 24 |
| III | 99 | 91 | 55 | 25 |
| IV | 100 | 97 | 69 | 47 |
| V | 100 | 94 | 57 | 11 |

### ANWENDUNGSBEISPIEL 4

### Impact der Kapseln mit Duftstoffmischungen I bis V

Eine handelsübliche parfümfreie Weichspülerzubereitung von Typ Vernell® wurde mit 0,01 Gew.-% der verkapselten Duftstoffmischungen I bis V versetzt. Anschließend wurden ca. 20 g dieser Mischung in einer handelsüblichen europäischen Haushaltswaschmaschine auf 2 kg Frotteehandtüchern verwaschen. Nach dem Schleudern wurden die Handtücher entnommen und getrocknet. Anschließend wurden die Handtücher unter mehrfachem Reiben von einem Panel bestehend aus 4 erfahrenen Testern gerieben und die Intensität der Geruchsentwicklung ("Impact") auf einer Skala von 1 (schwach) bis 10 (sehr stark) beurteilt. Die Ergebnisse sind in **Tabelle 5** zusammengefasst. Angegeben sind die Mittelwerte aus 3 aufeinanderfolgenden Testreihen.

**Tabelle 5**

| Impact | | | | |
|---|---|---|---|---|
| **Kapseln mit Duftstoffmischung** | **Geruchsintensität** | | | |
| | Tester 1 | Tester 2 | Tester 3 | Tester 4 |
| I | 8 | 7 | 7 | 8 |
| II | 7 | 6 | 7 | 7 |
| III | 8 | 8 | 7 | 5 |
| IV | 8 | 9 | 8 | 8 |
| V | 8 | 9 | 7 | 7 |

### FORMULIERUNGSBEISPIELE

**Tabelle 6**

| Stückseifen | | | |
|---|---|---|---|
| **Zusammensetzung** | **A1** | **A2** | **A3** |
| Sodium C12/14 Olefin Sulfonate | 40,0 | - | - |
| Disodium Lauryl Sulfosuccinate | - | 40,0 | - |
| Lauryl Glucoside | 15,0 | 15,0 | 55,0 |
| Cetylstearyl Alcohol | 12,0 | 12,0 | 12,0 |
| Paraffin Oil (Smp. 54 bis 56 °C) | 8,0 | 8,0 | 8,0 |
| Maze Starch, degradated | 8,0 | 8,0 | 8,0 |
| Palmitic Acid (and) Stearic Acid | 5,5 | 5,5 | 5,5 |
| Glyceryl Stearate | 2,0 | 2,0 | 2,0 |
| Coco Glyceride | 2,0 | 2,0 | 2,0 |
| Polyquaternium-7 | 1,0 | 1,0 | 1,0 |
| Parfümöl | 1,0 | 1,0 | 1,0 |
| Titandioxid | 0,5 | 0,5 | 0,5 |
| Kapseln mit Duftstoffmischung I | 0,5 | 0,5 | 0,5 |
| Wasser | ad 100 | | |

**Tabelle 7**

| Syndetseifen | | | |
|---|---|---|---|
| **Zusammensetzung** | **B1** | **B2** | **B3** |
| Lauryl Glucoside | 15,0 | 15,0 | 15,0 |
| Sodium C12/14 Olefin Sulfonate | 40,0 | 40,0 | - |
| Sodium Lauryl Sulfate | - | - | 40,0 |
| Paraffin Oil (Smp. 54 °C) | 8,0 | 8,0 | 8,0 |
| Tallow Fatty Alcohol | 7,0 | 7,0 | 7,0 |
| Maisstärke | 17,0 | - | 17,0 |
| Dextrose | - | 17,0 | - |
| Coco Fatty Acids | 10,0 | 10,0 | 10,0 |
| Titandioxid | 1,0 | 1,0 | 1,0 |
| Kapseln mit Duftstoffmischung II | 0,5 | - | 0,5 |
| Kapseln mit Duftstoffmischung III | - | 0,5 | - |
| Wasser | ad 100 | | |

**Tabelle 8**

| Gelförmiger Universalreiniger | | | | | | |
|---|---|---|---|---|---|---|
| **Zusammensetzung** | **C1** | **C2** | **C3** | **C4** | **C5** | **C6** |
| Lauryl Glucoside | 5,0 | 1,5 | 1,5 | 1,5 | 5,0 | 1,5 |
| Decyl Glucoside | - | 3,5 | 3,5 | 3,5 | - | 3,5 |
| Sodium Lauryl Sulfate | 9,0 | 6,0 | 3,0 | - | 9,0 | 6,0 |
| Sodium Laureth Sulfate | - | 3,0 | - | - | - | 3,0 |
| Cocamidopropyl Betaine | - | - | 6,0 | 9,0 | - | - |
| Ethanol | 3,5 | 3,5 | 3,5 | 3,5 | 3,5 | 3,5 |
| Citric Acid Mono Hydrate | 3,6 | 3,6 | 3,6 | 3,6 | 3,6 | 3,6 |
| Sodium Hydroxide | 0,5 | 0,5 | 0,5 | 0,5 | 0,5 | 0,5 |
| Laurylamin+1EO | 4,5 | 3,0 | 5,0 | 2,5 | - | - |
| Hexandiol-1,6 | - | 0,5 | - | | 0,5 | - |
| Kapseln mit Duftstoffmischung IV | 0,5 | - | 0,5 | - | 0,5 | - |
| Kapseln mit Duftstoffmischung V | - | 0,5 | - | 0,5 | - | 0,5 |
| Wasser | ad 100 | | | | | |

**Tabelle 9**

| Pulverwaschmittel | | | | | | |
|---|---|---|---|---|---|---|
| **Zusammensetzung** | **E1** | **E2** | **E3** | **E4** | **E5** | **E6** |
| Sodium Laureth-1 Sulfate | 12,0 | 8,0 | 8,0 | 4,0 | 4,0 | - |
| Dodecylbenzol Sulfonate | - | - | 4,0 | 8,0 | 4,0 | 8,0 |
| Sodium Lauryl Sulfate | - | 4,0 | - | - | 4,0 | 4,0 |
| Coco Fatty Acid, Sodium Salt | 1,0 | 1,0 | 1,0 | 1,0 | 1,0 | 1,0 |
| Coco Glucosides | - | - | - | 7,0 | - | - |
| Laureth-5 | 7,0 | 7,0 | 7,0 | - | 7,0 | 7,0 |
| Zeolith A | 24,0 | 24,0 | 24,0 | 24,0 | 24,0 | 24,0 |
| Natriumsilicat, amorph (Modul 1:2) | 3,0 | 3,0 | 3,0 | 3,0 | 3,0 | 3,0 |
| Natriumcarbonat | 12,0 | 12,0 | 12,0 | 12,0 | 12,0 | 12,0 |
| Polymeres Polycarboxylat | 5,5 | 5,5 | 5,5 | 5,5 | 5,5 | 5,5 |
| Perboratmonohydrat | 16,0 | 16,0 | 16,0 | 16,0 | 16,0 | 16,0 |
| Tetracetylethylendiamin | 5,5 | 5,5 | 5,5 | 5,5 | 5,5 | 5,5 |
| Carboxymethylcellulose | 0,3 | 0,3 | 0,3 | 0,3 | 0,3 | 0,3 |
| Optischer Aufheller | 0,2 | 0,2 | 0,2 | 0,2 | 0,2 | 0,2 |
| Enzymgranulat (Protease) | 1,0 | 1,0 | 1,0 | 1,0 | 1,0 | 1,0 |
| Kapseln mit Duftstoffmischung I | 0,5 | - | 0,5 | - | 0,5 | - |
| Kapseln mit Duftstoffmischung V | - | 0,5 | - | 0,5 | - | 0,5 |
| Wasser | ad 100 | | | | | |

**Tabelle 10**

| Handgeschirrspülmittel | | | | |
|---|---|---|---|---|
| **Zusammensetzung** | **F1** | **F2** | **F3** | **F4** |
| Sodium Laureth-1 Sulfate | 15 | 15 | 15 | 15 |
| Sodium Laureth-2 Sulfate | 15 | - | - | - |
| Sodium Lauryl Sulfate | - | 15 | - | - |
| Coco Glucosides | - | - | 15 | - |
| Sodium Octyl Sulfosuccinate | - | - | | 15 |
| Cocamidopropyl Betaine | 1 | 3 | 3 | 3 |
| Kapseln mit Duftstoffmischung III | 0,5 | - | 0,5 | - |
| Kapseln mit Duftstoffmischung IV | - | 0,5 | - | 0,5 |
| Wasser | ad 100 | | | |

**Tabelle 11**

| Reinigungsmittel | | | | | | |
|---|---|---|---|---|---|---|
| **Zusammensetzung** | **G1** | **G2** | **G3** | **G4** | **G5** | **G6** |
| C_{12/16}-Alkylsulfat-Na-Salz | 5,0 | 5,0 | 4,0 | 0 | 5,0 | 0 |
| C_{16/18}-Talgfettalkohol+25EO | 3,0 | 3,0 | 4,0 | 0 | 0 | 5,0 |
| C_{12/14}-Kokosalkyloligoglucosid | 1,0 | 2,0 | 2,0 | 5,0 | 0 | 0 |
| Natriummetasilicat | 15,0 | 15,0 | 15,0 | 15,0 | 15,0 | 15,0 |
| Natriumcarbonat | 26,0 | 24,0 | 24,0 | 30,0 | 30,0 | 30,0 |
| Sulfoniertes Styrol/MSA Copolymer, Natriumsalz | - | 2,0 | 2,0 | - | - | - |
| Kapseln mit Duftstoffmischung II | 0,5 | - | 0,5 | - | 0,5 | - |
| Kapseln mit Duftstoffmischung IV | - | 0,5 | - | 0,5 | - | 0,5 |
| Natriumtripolyphosphat | ad 100 | | | | | |

**Tabelle 12**

| Kosmetische Cremes | | | | | |
|---|---|---|---|---|---|
| **Zusammensetzung** | **H1** | **H2** | **H3** | **H4** | **H5** |
| Glyceryl Sterate (and) Ceteareth 12/20 (and) Cetearyl Alcohol (and) Cetyl Palmitate | 5,0 | 5,0 | 4,0 | - | - |
| Ceteareth-12 | - | - | 1,0 | - | - |
| Polyglyceryl-3 Isostearate | - | - | - | 4,0 | - |
| Polyglyceryl-2 Dipolyhydroxystearate | - | - | - | - | 4,0 |
| Glyceryl Oleate | - | - | - | 2,0 | - |
| PEG-7 Glyceryl Cocoate | - | - | - | - | 2,0 |
| Dicaprylyl Ether | - | - | - | 5,0 | 6,0 |
| Hexyldecanol (and) Hexyldecyl Laurate | - | - | 3,0 | 10,0 | 9,0 |
| Cetearyl Isononanoate | 3,0 | 3,0 | - | - | - |
| Decyl Oleate | 3,0 | 3,0 | - | - | - |
| Coco Caprylate Caprate | - | - | 3,0 | 5,0 | 5,0 |
| Bienenwachs | - | - | - | 7,0 | 5,0 |
| Hydrolyzed Elastin | 2,0 | - | - | - | - |
| Hydrolyzed Collagen | - | 2,0 | - | - | - |
| Hydrolyzed Wheat Gluten | - | - | 0,5 | - | - |
| Sodium Cocoyl Hydrolyzed Wheat Protein | - | - | - | 0,5 | 0,5 |
| Glycol Distearate (and) Laureth-4 (and) Cocamidopropyl Betaine | 5,0 | 5,0 | 4,0 | - | - |
| Glycerin (86 Gew.-%ig) | - | - | 1,0 | - | - |
| Kapseln mit Duftstoffmischung III | 0,5 | - | 0,5 | - | 0,5 |
| Kapseln mit Duftstoffmischung V | - | 0,5 | - | 0,5 | - |
| Wasser | ad 100 | | | | |

## Patentansprüche

1. Duftstoffmischung, enthaltend mindestens 60 Gew.-% Duftstoffe, die wenn sie entlang einer virtuellen Achse X, Y und Z ausgerichtet sind, eine Abmessung von X > 5 Å, Y> 3 Å und Z > 2 Å aufweisen,
wobei die Anzahl der so definierten verschiedenen Duftstoffen in der Duftstoffmischung mindestens zwei beträgt und die mindestens zwei Duftstoffe ausgewählt sind aus der Gruppe bestehend aus Agrunitril, Buccoblätteröl, Thiocineol, 2-Isobutyl-3-methoxypyrazin, Rosenoxid, Aldehyd C16, Methyloctincarbonat, Precyclemone B (CAS 52474-60-9), Allylamylglycolat, Cis-4-Decenal sowie deren Mischungen,
wobei mindestens einer der Duftstoffe in der Duftstoffmischung ein Schwefel- oder Stickstoffatom aufweist.

2. Duftstoffmischung nach Anspruch 1, **dadurch gekennzeichnet, dass** der Geruchsschwellenwert (GSW) der einzelnen Duftstoffmoleküle in der Mischung kleiner als 10 ppm ist.

3. Duftstoffmischung nach Anspruch 1 oder 2, **dadurch gekennzeichnet, dass** die Anzahl an verschiedenen Duftstoffen in der Mischung bei höchstens gleich oder unter 10 ist.

4. Duftstoffmischung nach einem der Ansprüche 1 bis 3, **dadurch gekennzeichnet, dass** mindestens eines der Duftstoffe in einer Konzentration von mindestens 10 Gew.-%, vorzugsweise höchstens 60 Gew.-%, in der Mischung vorliegt.

5. Duftstoffmischung nach einem der Ansprüche 1 bis 4, **dadurch gekennzeichnet, dass** mindestens einer der Duftstoffe in der Mischung ein Molgewicht von größer als 120 g/mol aufweist.

6. Duftstoffmischung nach einem der Ansprüche 1 bis 5, **dadurch gekennzeichnet, dass** die Duftstoffe in der Mischung einen log K_{ow} Wert von 1 bis 10 aufweisen.

7. Duftstoffmischung nach einem der Ansprüche 1 bis 6, **dadurch gekennzeichnet, dass** die Duftstoffmischung kein Lösungsmittel oder maximal 30 Gew.-% Lösungsmittel - bezogen auf die Duftstoffmischung - enthält.

8. Kapseln, enthaltend eine Duftstoffmischung nach einem der Ansprüche 1 bis 7.

9. Konsumprodukt, umfassend eine Duftstoffmischung nach einem der Ansprüche 1 bis 7 oder Kapseln nach Anspruch 8.

10. Konsumprodukt nach Anspruch 9, ausgewählt aus der Gruppe bestehend aus Wasch- und Reinigungsmitteln, kosmetischen Produkten, Parfümartikeln sowie kosmetischen Reinigungsmitteln.

11. Duftstoffmischung nach einem der Ansprüche 1 bis 7, **dadurch gekennzeichnet, dass** sie als Duftstoffe enthalten:
Agrunitril und Rosenoxid;
Agrunitril und Allylamylglycolat;
Agrunitril und cis-4-decenal;
Agrunitril und Diphenyloxid; oder
Agrunitril und Precyclemone B (CAS 52474-60-9).

12. Duftstoffmischung nach einem der Ansprüche 1 bis 7, **dadurch gekennzeichnet, dass** sie als Duftstoffe enthalten:
Buccoblätteröl und Allylamylglycolat;
Buccoblätteröl und cis-4-decenal; oder
Buccoblätteröl und Aldehyd C16

13. Duftstoffmischung nach einem der Ansprüche 1 bis 7, **dadurch gekennzeichnet, dass** sie als Duftstoffe enthalten:
Thiocineol und Rosenoxid; oder
Thiocineol und Precyclemone B (CAS 52474-60-9).

14. Duftstoffmischung nach einem der Ansprüche 1 bis 7, **dadurch gekennzeichnet, dass** sie als Duftstoffe enthalten:
2-Isobutyl-3-methoxypyrazin und Rosenoxid;
2-Isobutyl-3-methoxypyrazin und Allylamylglycolat;
2-Isobutyl-3-methoxypyrazin und cis-4-decenal; oder
2-Isobutyl-3-methoxypyrazin und Precyclemone B (CAS 52474-60-9).

## Claims

1. Fragrance mixture comprising at least 60 wt.-percent fragrances, showing dimensions of X > 5 Å, Y > 3 Å and Z > 2 Å when positioned along the virtual axes X, Y and Z,
wherein the number of different fragrances thus defined within said fragrance mixture is at least two and said at least two fragrances are selected from the group consisting of agrunitrile, bucco leaves oil, thiocineol, 2-isobutyl-3-methoxyparazin, rose oxide, aldehyde C16, methyloctincarbonate, precyclemone B (CAS 52474-60-9), allylamylglycolate, cis-4-decenal and mixtures thereof, and
wherein at least one of said fragrances in said fragrance mixture contains a sulphur or a nitrogen atom.

2. The fragrance mixture according to Claim 1, **characterized in that** the olfactory threshold (GSW) of each fragrance molecule in the mixture is below 10 ppm.

3. The fragrance mixture according to Claim 1 or 2, **characterized in that** the number of different fragrances in the mixture is at least equal or below 10.

4. The fragrance mixture according to any of Claims 1 to 3, **characterized in that** at least one fragrance is present in the mixture in a concentration of at least 10 wt.-percent, preferably at most 60 wt.-percent.

5. The fragrance mixture according to any of Claims 1 to 4, **characterized in that** at least one fragrance in the mixture shows a molecular weight of above 120 g/mol.

6. The fragrance mixture according to any of Claims 1 to 5, **characterized in that** the fragrances in the mixture shows a log K_{OW} value of 1 to 10.

7. The fragrance mixture according to any of Claims 1 to 6, **characterized in that** the fragrance mixture does not contain any solvent or at most 30 wt.-percent solvent - calculated on the fragrance mixture.

8. Capsules, comprising a fragrance mixture according to any of Claims 1 to 7.

9. Consumer product, comprising a fragrance mixture according to any of Claims 1 to 7 or capsules according to Claim 8.

10. Consumer product according to Claim 9, selected from the group consisting of washing and cleaning compositions, cosmetic products, perfume articles and cosmetic cleaners.

11. The fragrance mixture according to any of Claims 1 to 7, **characterized in that** they comprise as fragrances:
agrunitrile and rose oxide;
agrunitrile and cis-4-decenal;
agrunitrile and diphenyloxide; or
agrunitrile and precyclemone B (CAS 52474-60-9).

12. The fragrance mixture according to any of Claims 1 to 7, **characterized in that** they comprise as fragrances:
bucco leaves oil an allylamylglycolate:
bucco leaves oil and cis-4-decenal; or
bucco leaves oil and aldehyde C16.

13. The fragrance mixture according to any of Claims 1 to 7, **characterized in that** they comprise as fragrances:
thiocineol and rose oxide; or
thiocineol and precyclemone B (CAS 52474-60-9).

14. The fragrance mixture according to any of Claims 1 to 7, **characterized in that** they comprise as fragrances:
2-isobutyl-3-methoxyparzin and rose oxide;
2-isobutyl-3-methoxyparzin and allylamylglycolate;
2-isobutyl-3-methoxyparzin and cis-4-decenal; or
2-isobutyl-3-methoxyparzin and precyclemone B (CAS 52474-60-B).

## Revendications

1. Mélange de parfums, contenant au moins 60 % en poids de parfums, qui présentent lorsqu'ils sont alignés le long d'un axe virtuel X, Y et Z une dimension X > 5 Å, Y > 3 Å et Z > 2 Å,
le nombre de parfums différents ainsi définis dans le mélange de parfums étant d'au moins deux, et lesdits au moins deux parfums étant choisis dans le groupe constitué par l'agrunitrile, l'huile de feuille de bucco, le thiocinéol, la 2-isobutyl-3-méthoxypyrazine, l'oxyde de rose, l'aldéhyde C16, le carbonate de méthyloctine, le précyclémone B (CAS 52474-60-9), le glycolate d'allylamyle, le cis-4-décénal et leurs mélanges,
au moins un des parfums dans le mélange de parfums comprenant un atome de soufre ou d'azote.

2. Mélange de parfums selon la revendication 1, **caractérisé en ce que** la valeur de seuil olfactif (GSW) des molécules de parfum individuelles dans le mélange est inférieure à 10 ppm.

3. Mélange de parfums selon la revendication 1 ou 2, **caractérisé en ce que** le nombre de parfums différents dans le mélange est au plus égal ou inférieur à 10.

4. Mélange de parfums selon l'une quelconque des revendications 1 à 3, **caractérisé en ce qu'**au moins un des parfums est présent dans le mélange en une concentration d'au moins 10 % en poids, de préférence d'au plus 60 % en poids.

5. Mélange de parfums selon l'une quelconque des revendications 1 à 4, **caractérisé en ce qu'**au moins un des parfums dans le mélange présente un poids moléculaire supérieur à 120 g/mol.

6. Mélange de parfums selon l'une quelconque des revendications 1 à 5, **caractérisé en ce que** les parfums dans le mélange présentent une valeur log K_{ow} de 1 à 10.

7. Mélange de parfums selon l'une quelconque des revendications 1 à 6, **caractérisé en ce que** le mélange de parfums ne contient pas de solvant ou au plus 30 % en poids de solvant, par rapport au mélange de parfums.

8. Capsules, contenant un mélange de parfums selon l'une quelconque des revendications 1 à 7.

9. Produit de consommation, comprenant un mélange de parfums selon l'une quelconque des revendications 1 à 7 ou des capsules selon la revendication 8.

10. Produit de consommation selon la revendication 9, choisi dans le groupe constitué par les détergents, les produits cosmétiques, les articles de parfumerie, ainsi que les détergents cosmétiques.

11. Mélange de parfums selon l'une quelconque des revendications 1 à 7, **caractérisé en ce qu'**il contient en tant que parfums :
de l'agrunitrile et de l'oxyde de rose ;
de l'agrunitrile et du glycolate d'allylamyle ;
de l'agrunitrile et du cis-4-décénal ;
de l'agrunitrile et de l'oxyde de diphényle ; ou
de l'agrunitrile et de la précyclémone B (CAS 52474-60-9).

12. Mélange de parfums selon l'une quelconque des revendications 1 à 7, **caractérisé en ce qu'**il contient en tant que parfums :
de l'huile de feuille de bucco et du glycolate d'allylamyle ;
de l'huile de feuille de bucco et du cis-4-décénal ; ou
de l'huile de feuille de bucco et de l'aldéhyde C16.

13. Mélange de parfums selon l'une quelconque des revendications 1 à 7, **caractérisé en ce qu'**il contient en tant que parfums :
du thiocinéol et de l'oxyde de rose ; ou
du thiocinéol et de la précyclémone B (CAS 52474-60-9).

14. Mélange de parfums selon l'une quelconque des revendications 1 à 7, **caractérisé en ce qu'**il contient en tant que parfums :
de la 2-isobutyl-3-méthoxypyrazine et de l'oxyde de rose ;
de la 2-isobutyl-3-méthoxypyrazine et du glycolate d'allylamyle ;
de la 2-isobutyl-3-méthoxypyrazine et du cis-4-décénal ; ou
de la 2-isobutyl-3-méthoxypyrazine et de la précyclémone B (CAS 52474-60-9).
